# EUROPEAN PATENT APPLICATION

(11) **EP 2 103 263 A1**
(43) Date of publication of application: **23.09.2009**
(21) Application number: 09003324.2
(22) Date of filing: 06.03.2009
(51) Int. Cl.: A61B 17/221, A61F 2/01, A61B 17/22, A61B 19/00

(54) **Shaft for operating a blood thrombus capturing member, and blood thrombus capturing catheter**

(30) Priority: 21.03.2008 JP 2008072777
(71) Applicant: Nipro Corporation, Kita-ku Osaka-shi, Osaka 531-8510 (JP)
(72) Inventor: Miyagawa, Katsuya, Osaka-shi Osaka 531-8510 (JP); Umegaki, Ryota, Osaka-shi Osaka 531-8510 (JP); Kakinoki, Misa, Osaka-shi Osaka 531-8510 (JP)
(74) Representative: Schmitz, Hans-Werner

(57) **Abstract**

A shaft for operating a blood thrombus capturing member (2), comprises a blood thrombus capturing member (3) equipped on the distal end side thereof, and a guide tip (4) quipped at the distal end, through which a guide wire is inserted so as to be capable of relative movement, wherein the shaft (2) is flexible. Therefore, the distal end component of a blood thrombus capturing catheter can be easily guided to a vein constriction, and particularly with a branched vein, the distal end component of the blood thrombus capturing catheter can be easily guided to the targeted branch of the vein.

## Description

### BACKGROUND OF THE INVENTION

### 1. Field of the Invention

The present invention relates to a shaft for operating a blood thrombus capturing member, and to a blood thrombus capturing catheter.

### 2. Background Information

Stents, POBA (Plain Old Balloon Atherectomy), and other such percutaneous transluminal angioplasty techniques were used in the past for angiostenosis of the coronary artery, the carotid artery, venous grafts, etc. These techniques are a type of revascularization for ensuring blood flow, in which stent placement or balloon inflation is performed at the site of angiostenosis, and these have been productive.

Nevertheless, while the above technique does expand the constriction, any blood thrombus, plaque (protuberant lesions), or the like that may have been loosened during the procedure may flow to the peripheral side of the lesions and create infarction, no-reflow, or other such problems, and reflow could not be obtained. Complications caused by these blood thrombus and so forth are often serious.

For example, with unstable angina, acute myocardial infarction, or another such disorder of the coronary artery in which blood thrombus are involved, the thrombus is pushed out by stent placement or balloon inflation, and veins on the peripheral side of the lesions are often blocked off. As a result, not enough oxygen and nutrients are supplied to the heart muscles, leading to myocardial disturbance such as myocardial necrosis. Similarly, if a vein supplying blood to the brain becomes clogged, this leads to what is known as cerebral infarction, resulting in a state in which the brain tissue is dead.

The carotid artery is particularly susceptible to constriction due to arteriosclerosis, which can result in insufficient blood flow to the brain, and cerebral infarction tends to be caused by blood thrombus produced by constriction. Symptoms of such cerebral infarction include paralysis, numbness, aphasia, clouding of consciousness, and so forth, and once lost, a function cannot be expected to be recovered 100% even through rehabilitation, and this has a serious impact on the subsequent lifestyle of the patient. Also, large quantities of blood thrombus adhere to old, modified vein graft constrictions, so care is required.

Since infarction caused by blood thrombus thus leads to serious complications, the peripheral side of lesions must be protected in percutaneous transluminal angioplasty. With this in mind, the following blood thrombus capturing catheter has already been proposed (see Japanese Laid-Open Patent Application 2004-97807, for example).

Such blood thrombus capturing catheter has a blood thrombus capturing member delivery sheath, a shaft for operating a blood thrombus capturing member, and a blood thrombus capturing member. The shaft is flexible, is inserted into the delivery sheath so as to be able to move in the axial direction, and protrudes forward from the distal end of the sheath. The blood thrombus capturing member is provided to the distal end component of the shaft, is housed inside the distal end component of the delivery sheath, and by operation of the shaft, the entire member protrudes forward in the axial direction of the sheath and, when thus protruding, expands outward in the radial direction and contracts in the axial direction through elastic deformation.

This blood thrombus capturing catheter is inserted into a vein, and its distal end component is guided to the targeted constriction of the vein, but in the past, no means was provided for guiding the distal end component of this blood thrombus capturing catheter to the targeted constriction of the vein. Accordingly, it was not easy to guide the distal end component of the blood thrombus capturing catheter to the targeted constriction of the vein, and particularly when the vein branched, it was difficult to guide the distal end component of the blood thrombus capturing catheter to the targeted branch of the vein.

### SUMMARY OF THE INVENTION

It is an object of the present invention to provide a shaft for operating a blood thrombus capturing member with which the distal end component of a blood thrombus capturing catheter can be easily guided to the constricted part of a vein, as well as a blood thrombus capturing catheter.

The present invention provides a shaft for operating a blood thrombus capturing member, comprising
a blood thrombus capturing member equipped on the distal end side thereof, and a guide tip equipped at the distal end, through which a guide wire is inserted so as to be capable of relative movement,
wherein the shaft is flexible.
Further, the present invention provides a blood thrombus capturing catheter, comprising:
a blood thrombus capturing member delivery sheath;
the shaft for operating a blood thrombus capturing member according to Claim 1; and
a blood thrombus capturing member that is housed in the distal end of the sheath and that protrudes forward in the axial direction of the sheath by the operation of the shaft and expands outward in the radial direction and contracts in the axial direction through elastic deformation.

With the present invention, the distal end component of a blood thrombus capturing catheter can be easily guided to a vein constriction, and particularly with a branched vein, the distal end component of the blood thrombus capturing catheter can be easily guided to the targeted branch of the vein.

### BRIEF DESCRIPTION OF THE DRAWINGS

FIG. 1 is a longitudinal cross-sectional view of the blood thrombus capturing catheter of the first embodiment pertaining to the present invention.
FIG. 2 is a longitudinal cross-sectional view of the state when the blood thrombus capturing member expands illustrating the blood thrombus capturing catheter of FIG. 1.
FIG. 3 is a longitudinal cross-sectional view of the using state illustrating the blood thrombus capturing catheter of FIG. 1.
FIG. 4 is a longitudinal cross-sectional view of the using state illustrating the blood thrombus capturing catheter of FIG. 1.
FIG. 5 is a longitudinal cross-sectional view of the using state illustrating the blood thrombus capturing catheter of FIG. 1.
FIG. 6 is a longitudinal cross-sectional view of the blood thrombus capturing catheter of the second embodiment pertaining to the present invention.
FIG. 7 is a longitudinal cross-sectional view of the blood thrombus capturing catheter of the third embodiment pertaining to the present invention.
FIG. 8 is a longitudinal cross-sectional view of the blood thrombus capturing catheter of the fourth embodiment pertaining to the present invention.
FIG. 9 is a transverse cross-sectional view along the line A-A of the blood thrombus capturing catheter of the FIG. 8 in the direction of the arrow.
FIG. 10 is a longitudinal cross-sectional view of the blood thrombus capturing catheter of the fifth embodiment pertaining to the present invention.

### DETAILED DESCRIPTION OF THE PREFERRED EMBODIMENTS

### Embodiment 1

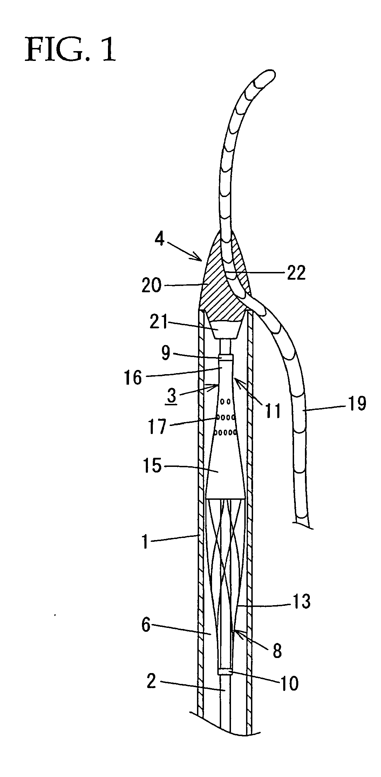

One preferred embodiment of a blood thrombus capturing catheter of the present invention will now be described in detail through reference to FIGS. 1 to 5.
The blood thrombus capturing catheter comprises a blood thrombus capturing member delivery sheath 1, a shaft for operating a blood thrombus capturing member 2, a blood thrombus capturing member 3 and a guide tip 4.

The sheath I is a tube-shaped member that is flexible, and a lumen 6 that is open at the distal end extends (substantially) the entire length, from the proximal end to the distal end. The sheath 1 needs to be flexible and biocompatible, so it is formed, for example, from polyurethane, polyethylene, polyester, polypropylene, polyamide, polytetrafluoroethylene, polyvinylidene fluoride, or another such synthetic resin.

The shaft 2 operates the blood thrombus capturing member 3, is a linear member that is flexible and pliant, is inserted into the lumen 6 of the sheath 1 so as to be capable of movement (back and forth) in the axial direction, and sticks out from the sheath 1 forward and rearward in the axial direction. The shaft 2 is formed from stainless steel, a nickel-titanium alloy, or another such metal material. A guide wire that is used in percutaneous transluminal angioplasty is used as the shaft 2.

The blood thrombus capturing member 3 is provided to the distal end component of the shaft 2, captures blood thrombus, plaque, and so forth, is entirely housed within the distal end component of the sheath 1, entirely protrudes forward in the axial direction from the sheath 1 by operation of the sheath 1, and when protruding, the blood thrombus capturing member 3 expands outward in the radial direction and contracts in the axial direction through elastic deformation. There are no particular restrictions on the outside diameter of the blood thrombus capturing member 3, but it may be 3.0 mm, 3.5 mm, 4.0 mm, 4.5 mm, 5.0 mm, 5.5 mm, 6.0 mm, 6.5 mm, 7.0 mm, 7.5 mm, 8.0 mm, for example, and the outside diameter is preferably selected according to the inside diameter of the vein at the lesion, that is, the vein constriction. The blood thrombus capturing member 3 has a wire member 8, a slide ring 9, a fixed ring 10, and a filter 11.

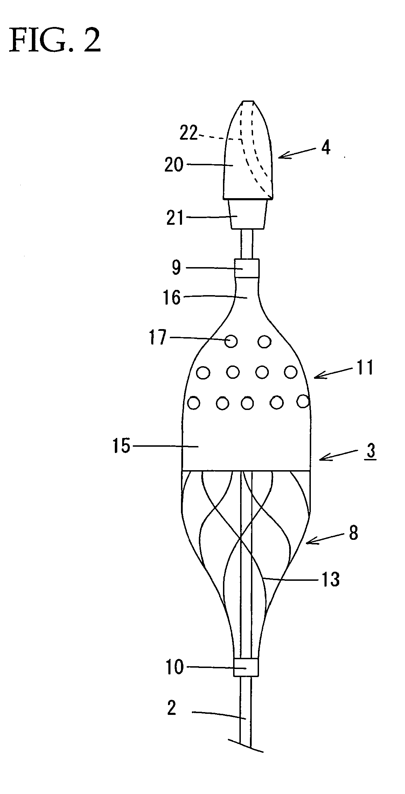

The wire member 8 is substantially spindle-shaped, around which a plurality of wires 13 are wound in a spiral, whose distal end and proximal end are constricted inward in the radial direction, and whose middle component bulges outward in the radial direction. The material from which the wires 13 are formed can be a shape memory alloy, and an Ni-Ti alloy, a CuZn-Al alloy, or the like is generally employed. When a shape memory alloy is used as the material that forms the wires 13, the wire member 8 is usually held in the shape shown in FIG. 2 (restored shape, original shape). When the blood thrombus capturing member 3 is used for a lesion with a large quantity of blood thrombus, etc., the wire member 8 is sometimes formed longer in order to capture more blood thrombus, etc. The number of wires 13 that make up the wire member 8 may be from 8 to 16, and preferably from 8 to 14, and ideally from 8 to 12.

The slide ring 9 is fitted externally to the distal end component of the shaft 2 so as to be able to move (slide) in the axial direction, and the distal end of the wire member 8 is fixed to the slide ring 9. The fixed ring 10 is fitted and fixed externally more to the proximal end side than the slide ring 9 at the distal end component of the shaft 2, and the proximal end of the wire member 8 is affixed. The rings 9 and 10 are formed from stainless steel or another such metal material.

The filter 11 is formed in a funnel shape from a nonwoven thrombus or a knitted fabric composed of polyurethane, polyethylene, polyester, polypropylene, polyamide, polytetrafluoroethylene, polyvinylidene fluoride, or another such synthetic resin, for example (a biocompatible material), and consists of a bulging component 15 on the proximal end side and a constricted component 16 on the distal end side. A plurality of holes 17 that are small enough to capture blood thrombus and so forth, but are large enough to allow bodily fluids to pass through, are provided at the portion where there is a transition from the bulging component 15 to the constricted component 16. The filter 11 covers approximately half of the wire member 8 on the distal end component side, including the bulging middle portion (excluding the portion of the wire member 8 fixed by the slide ring 9), the distal end of the constricted component 16 is adjacent to the slide ring 9, and at least the bulging component 15 is bound and fixed with yarn or the like to the wire member 8. The filter 11 may be formed by dipping to the restored shape of the wire member 8, after which the above-mentioned holes 17 are made, and the wire member 8 then covered. The diameter of the holes 17 is from 50 to 1000 µm, and preferably from 50 to 500µm, and ideally from 100 to 200µm. If the diameter of the holes 17 is too small, blood flow tends to be impeded, but if the holes are too large, there will be no point in providing the filter 11. In the case of a large blood thrombus, the filter 11 may not be provided to the blood thrombus capturing member 3, and the blood thrombus capturing member 3 may instead comprise the wire member 8, the slide ring 9, and the fixed ring 10, so that the blood thrombus is captured by the openings in the wire member 8.

The guide tip 4 is flexible, is affixed to the distal end of the shaft 2, has a guide wire 19 inserted therein so as to be capable of relative movement, and guides a blood thrombus capturing catheter through joint action with the guide wire 19. This guide tip 4 has a lateral cross section that is circular, and is the result of integrally forming a distal end constituting component 20 on the distal end side and a proximal end constituting component 21 on the proximal end side. In the example depicted in the drawings, the shaft 2 does not protrude from the distal end of the guide tip 4, but the shaft 2 may also protrude from the distal end of the guide tip 4. The distal end constituting component 20 constitutes the distal end side of the guide tip 4, is contiguous with the proximal end constituting component 21 in the axial direction, and the two components 20, 21 are coaxial. The distal end constituting component 20 decreases in diameter toward its distal end. The outside diameter of the sheath 1 is (substantially) the same as that of the proximal end of the distal end constituting component 20, and when the blood thrombus capturing member 3 is housed in the sheath 1, the proximal end face of the distal end constituting component 20 comes into contact with the distal end face of the sheath, so that the distal end opening of the sheath 1 is closed off by the distal end constituting component 20. As discussed above, when the blood thrombus capturing member 3 is housed in the sheath 1, the proximal end face of the distal end constituting component 20 comes into contact with the distal end face of the sheath, which prevents the guide tip 4 from being pulled too far into the sheath 1, so there is no risk that the guide wire 19 inserted into the guide tip 4 will be pinched within the sheath 1. An insertion hole 22, which opens at the distal end and the side face of the proximal end and into which is inserted the guide wire 19, is formed in the distal end constituting component 20. The insertion hole 22 is slanted with respect to the axis, and is also curved with respect to the axis. The proximal end constituting component 21 constitutes the proximal end side of the guide tip 4, has a smaller diameter than the proximal end of the distal end constituting component 20, is stepped with respect to the distal end constituting component 20, has a truncated conical shape, and its diameter decreases toward the proximal end. The diameter of the proximal end constituting component 21 is smaller than the inside diameter of the sheath 1, that is, the diameter of the lumen 6, and when the blood thrombus capturing member 3 is housed in the sheath 1, the proximal end constituting component 21 is inserted into the distal end component of the sheath 1. The guide tip 4 may be flexible and biocompatible, so it may be formed from the same material as the sheath 1. The maximum outside diameter of the guide tip 4 may be about 8.0 to 2 mm and the inside diameter of the insertion hole 22 may be about 0.4 to 1.2 mm.

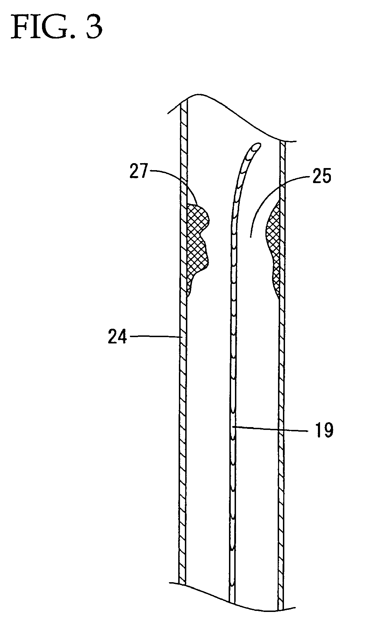

It will now describe percutaneous transluminal angioplasty in which the blood thrombus capturing catheter of the present invention is used to remove blood thrombus and plaque from a vein constriction.
First, the guide wire 19 is inserted ahead of time into a vein 24, and as shown in FIG. 3, the distal end component of the guide wire 19 is passed through a vein constriction 25 in the targeted vein 24.

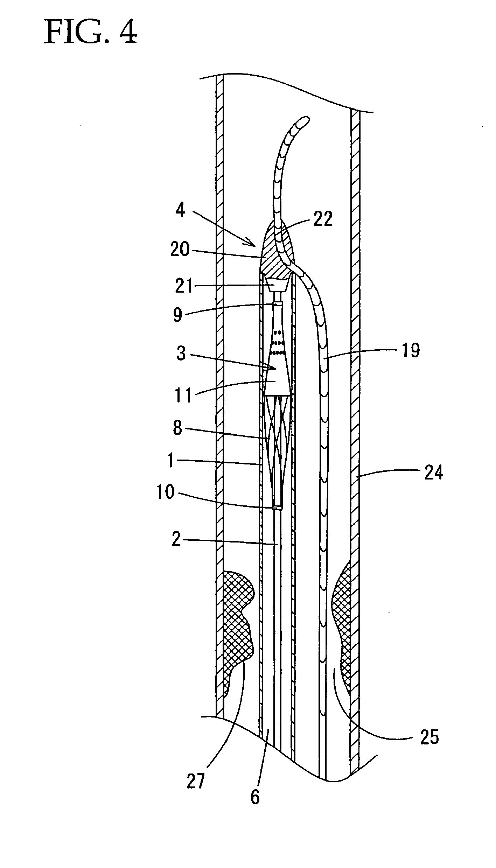

Next, the proximal end component of the guide wire 19 is inserted in the insertion hole 22 of the guide tip 4 of the blood thrombus capturing catheter, the blood thrombus capturing catheter is inserted into the vein 24, the guide tip 4 is slid along the guide wire 19 by being guided by the guide wire 19, and the guidance provided by the joint action of the guide wire 19 and the guide tip 4 introduces the distal end component of the blood thrombus capturing catheter into the targeted vein 24, and allows it to pass through the vein constriction 25 in the vein 24 as shown in FIG. 4.

Thus, the blood thrombus capturing catheter can be introduced into the targeted vein 24 by the guidance provided by the joint action of the guide wire 19 and the guide tip 4, so the distal end component of the blood thrombus capturing catheter can be easily guided to the targeted vein constriction 25, and particularly with the branched vein, the distal end component of the blood thrombus capturing catheter can be easily guided to the targeted branch of the vein.

Also, the distal end constituting component 20, which is on the outside of the sheath 1 at the guide tip 4 when the blood thrombus capturing member 3 is housed in the sheath 1, decreases in diameter toward its distal end, so when the blood thrombus capturing catheter is moved in the vein, the blood thrombus capturing catheter encounters less movement resistance from blood.

Further, when the blood thrombus capturing member 3 is housed in the sheath 1, the proximal end face of the distal end constituting component 20 comes into contact with the distal end face of the sheath 1, so that the distal end opening of the sheath 1 is closed off by the distal end constituting component 20. Therefore, when the blood thrombus capturing catheter moves through the vein, no blood at all, or almost no blood, gets into the sheath 1.

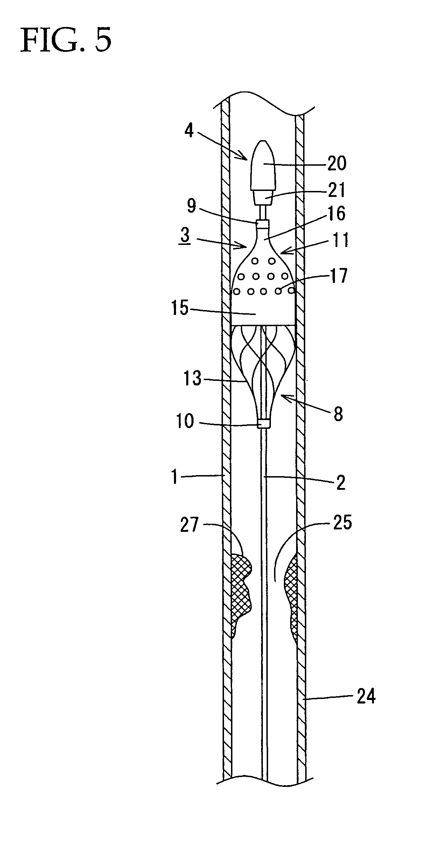

As discussed above, after the distal end component of the blood thrombus capturing catheter has passed through the vein constriction 25 of the vein 24, when the sheath 1 is pulled rearward in the axial direction (toward the front, the proximal end side) in a state of the shaft 2 being fixed, then as shown in FIG. 5, the entire blood thrombus capturing member 3 protrudes outside the sheath 1, expands (enlarges) outward in the radial direction and constricts in the axial direction through elastic deformation, and is pressed snugly against the inner wall of the vein 24.

Then, the shaft 2 and the blood thrombus capturing member 3 are left inside the vein 24, while just the sheath 1 is taken out of the patient's body, a balloon catheter (not shown) that has been readied separately is externally fitted to the shaft 2, the balloon thereof (not shown) is inserted up to the vein constriction 25 and inflated with a pressure medium, so that the blood thrombus, plaque, or other adhering material comes loose from the vein constriction 25 and is captured by the blood thrombus capturing member 3. After this, the pressure medium is released from the balloon, the balloon is deflated, and just the balloon catheter is taken out of the body.

After this, a blood thrombus capturing member recovery sheath (not shown) that is larger in diameter than the delivery sheath 1 is externally fitted to the shaft 2, and its distal end component is inserted up to in front of the vein constriction 25, after which the recovery sheath is pushed forward in the axial direction (the distal end side of the shaft 2) in a state of the shaft 2 being fixed. Consequently, the proximal end side of the blood thrombus capturing member 3 comes into contact with the distal end of the recovery sheath, and the blood thrombus capturing member 3 shrinks inward in the radial direction and elongates in the axial direction through elastic deformation, so that the proximal end component of the bulging component 15 of the filter 11 and the proximal end side of the wire member 8, that is, the proximal end side of the blood thrombus capturing member 3, are housed in the distal end component of the recovery sheath.

Since just the proximal end component of the bulging component 15 of the filter 11 and the proximal end side of the wire member 8, that is, the proximal end side of the blood thrombus capturing member 3, are housed in the distal end component of the recovery sheath, there is no risk that the blood thrombus capturing member 3 will be compressed too much, and that the blood thrombus, etc., held in the blood thrombus capturing member 3 will lead out from the holes 17 in the filter 11.

After the proximal end side of the blood thrombus capturing member 3 has been housed in the distal end component of the recovery sheath as discussed above, the recovery sheath is taken out of the body along with the shaft 2 and the blood thrombus capturing member 3 to complete the percutaneous angioplasty on the vein 24.

### Embodiment 2 and 3

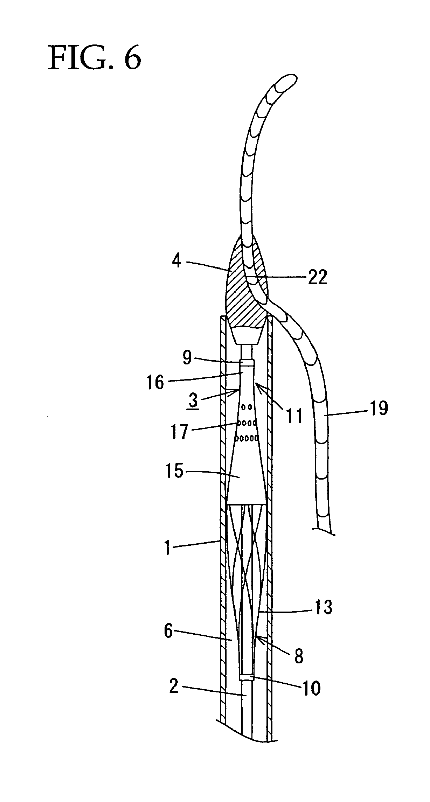

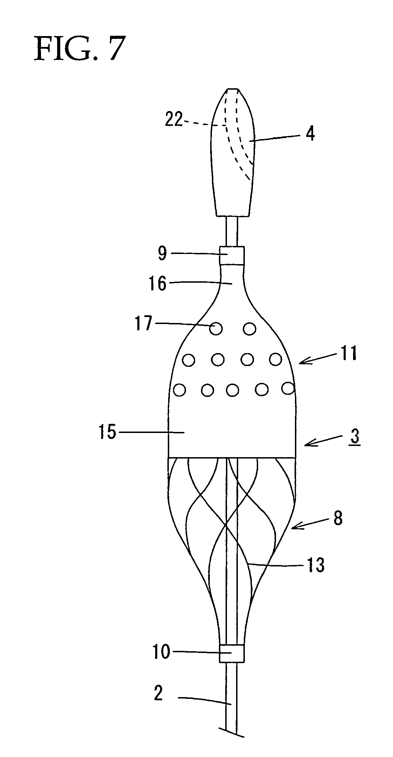

FIGS. 6 and 7 illustrate second and third embodiments of the present invention, in which the guide tip 4 is substantially spindle-shaped (shaped substantially like a rugby ball) or a similar shape, and the largest diameter portion thereof is disposed in the approximate center in the axial direction of the guide tip or on the proximal end side. If the above-mentioned largest diameter portion of the guide tip 4 is larger than the inside diameter of the blood thrombus capturing member delivery sheath 1, then when the blood thrombus capturing member 3 is housed in the sheath 1, this largest diameter portion will come into contact with the distal end of the sheath 1, that is, a side face of the distal end constituting component of the guide tip comes into contact with a inside face on the distal end side of the sheath, so that the guide tip 4 is prevented from being pulled too far into the sheath 1 and the distal end opening of the sheath 1 can be closed off.

### Embodiment 4 and 5

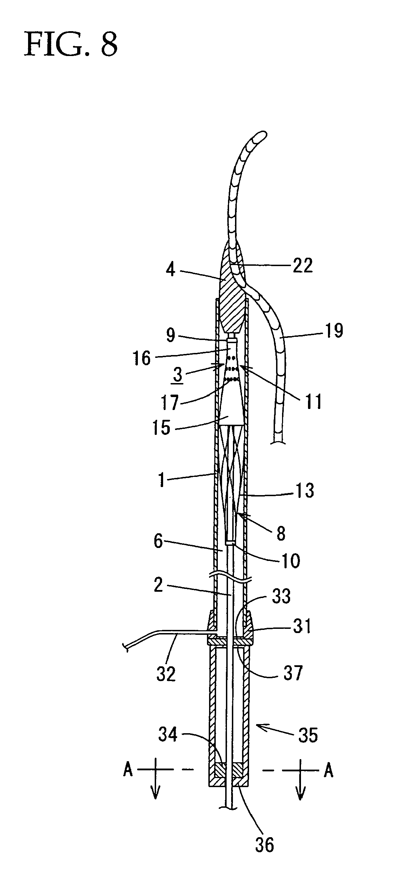

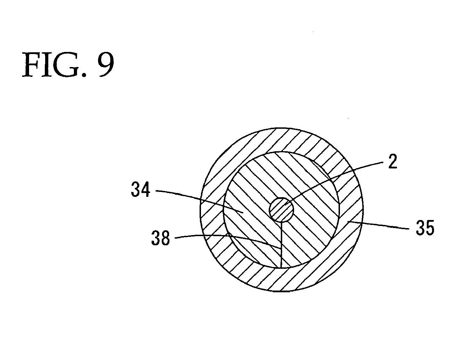

In the second and third embodiments of the present invention, if the above-mentioned largest diameter portion of the guide tip 4 is smaller than the inside diameter of the blood thrombus capturing member delivery sheath 1, then when the blood thrombus capturing member 3 is housed in the sheath 1, there is the risk that the guide tip 4 will be pulled too far into the sheath 1. FIGS. 8 and 9 illustrate fourth and fifth embodiments of the present invention that solve this problem.

In the fourth embodiment of the present invention shown in FIGS. 8 and 9, a bottomed, cylindrical blocking member 31 that blocks off the proximal end opening of the blood thrombus capturing member delivery sheath 1 is externally fitted and fixed to the proximal end component of this sheath 1, and the shaft 2 is inserted in the blocking member 31. The blocking member 31 is formed from polypropylene, an ABS resin, polyvinyl chloride, polyethylene, polyethylene terephthalate, or another such synthetic resin, or from stainless steel, brass, or another such metal material. The blocking member 31 is connected to a side-filling tube 32, so that heparin or the like can be injected into the sheath 1 to prevent the blood from coagulating. A blood stop valve 33 is inserted into the proximal end component of the blocking member 31, which prevents blood from leaking out from the insertion portion of the shaft 2 at the blocking member 31. A ring-shaped contact component 34 composed of an elastic material is externally fitted to the proximal end of the shaft 2. The contact component 34 cannot move in the axial direction with respect to the shaft 2, but a slit 38 extending from the outer face to the inner face is formed along the entire length of the contact component 34 in the axial direction, and the shaft 2 is able to remove outward and away from the contact component 34 via the slit 38 through the elastic deformation of the contact component 34. A stopper 35 is affixed to the proximal end face of the blocking member 31, and is contiguous in the axial direction. The stopper 35 is cylindrical in shape, closed off at both ends in the axial direction, the proximal end of the shaft 2 is inserted removably and movably in the axial direction, and the contact component 34 is installed movably in the axial direction. The proximal end component of the stopper 35 comes into contact with the contact component 34, creating a rearward movement limiter 36 that limits rearward movement of the shaft 2 in the axial direction, and the distal end component of the stopper 35 comes into contact with the contact component, creating a forward movement limiter 37 that limits forward movement of the shaft in the axial direction.

With the embodiments constituted as above, when the blood thrombus capturing member 3 is housed in the sheath 1, rearward movement of the shaft 2 in the axial direction is limited by the joint action of the rearward movement limiter 36 of the stopper 35 and the contact component 34 provided to the shaft 2, so that the guide tip 4 is prevented from being pulled too far into the sheath 1, which means that there is no risk that the guide wire 19 inserted into the guide tip 4 will be pinched within the sheath 1. Also, when the sheath 1 is pulled forward in the axial direction (to the front side, the proximal end side), and the blood thrombus capturing member 3 is made to protrude outward from the sheath 1, the joint action of the forward movement limiter 37 of the stopper 35 and the contact component 34 limits forward movement of the shaft 2 in the axial direction, so the blood thrombus capturing member 3 can be easily kept at the proper distance from the distal end of the sheath 1. Furthermore, when the delivery sheath 1 is replaced with a blood thrombus capturing member recovery sheath, the sheath 1 and the stopper 35 can be easily removed from the shaft 2 by removing the shaft 2 from the contact component 34 to the outside via the slit 38.

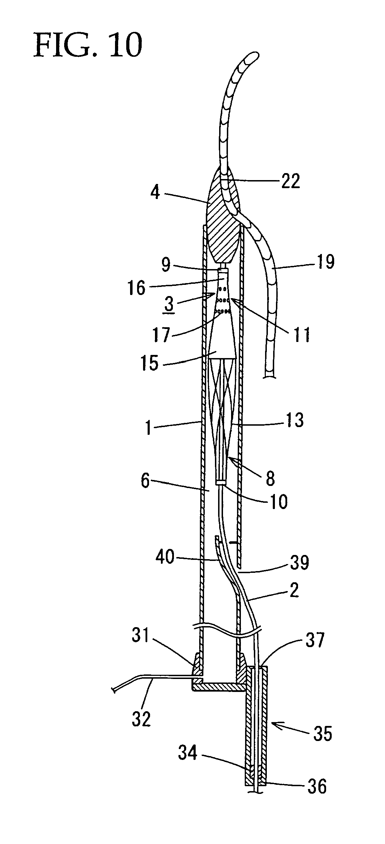

In the fifth embodiment of the present invention shown in FIG. 10, a side hole 39 through which the shaft 2 can be inserted is formed in the side wall of the distal end component of the sheath 1, and a dividing wall 40 is formed inside the distal end component of the sheath 1. The interior of this dividing wall 40 communicates with the distal end side of the shaft 2 and the side hole 39, and the shaft 2 is inserted into said interior.

With the distal end constituting component that constitutes the distal end side of the guide tip, and is smaller in diameter on the distal end than the proximal end, so the blood thrombus capturing catheter is subjected to less movement resistance from the blood when the blood thrombus capturing catheter is moved through a vein and its distal end component is guided to the targeted vein constriction.
Also, when the blood thrombus capturing member is housed in the blood thrombus capturing member delivery sheath, the proximal end face of the proximal end constituting component of a guide tip comes into contact with the distal end face of the sheath, which prevents the guide tip from being pulled too far into the sheath, so there is no risk that the guide wire inserted into the guide tip will be pinched within the sheath. Further, when the blood thrombus capturing member is housed in the blood thrombus capturing member delivery sheath, moved through the vein, and its distal end component guided to the targeted vein constriction, the blood thrombus capturing member is housed within the sheath, but since the distal end opening of the sheath is blocked off by the distal end constituting component of the guide tip when this housing is in progress, no blood at all, or almost no blood, gets into the sheath during the above-mentioned movement.
Moreover, when the blood thrombus capturing member is housed in the blood thrombus capturing member delivery sheath, the joint action of the rearward movement limiter and the contact component provided to the shaft limits rearward movement of the shaft in the axial direction, and prevents the guide tip from being pulled too far into the sheath, so there is no risk that the guide wire inserted into the guide tip will be pinched within the sheath.
Further more, when the sheath is pulled rearward in the axial direction so that the blood thrombus capturing member sticks out from the sheath, the joint action of the contact component and the forward movement limiter limit the forward movement of the shaft in the axial direction, so that the blood thrombus capturing member can easily be kept a suitable distance away from the distal end of the sheath.

This application claims priority to Japanese Patent Application No. 2008-072777. The entire disclosure of Japanese Patent Application No. 2008-072777 is hereby incorporated herein by reference.
While only selected embodiments have been chosen to illustrate the present invention, it will be apparent to those skilled in the art from this disclosure that various changes and modifications can be made herein without departing from the scope of the invention as defined in the appended claims. Furthermore, the foregoing descriptions of the embodiments according to the present invention are provided for illustration only, and not for the purpose of limiting the invention as defined by the appended claims and their equivalents. Thus, the scope of the invention is not limited to the disclosed embodiments.

## Claims

1. A shaft for operating a blood thrombus capturing member (2), comprising
a blood thrombus capturing member (3) equipped on the distal end side thereof, and a guide tip (4) equipped at the distal end, through which a guide wire is inserted so as to be capable of relative movement,
wherein the shaft (2) is flexible.

2. The shaft according to Claim 1, wherein the guide tip (4) has:
a distal end constituting component (20) that constitutes the distal end side of the guide tip (4), and is smaller in diameter on the distal end than the proximal end; and
a proximal end constituting component (21) that constitutes the proximal side of the guide tip (4) and is smaller in diameter than the proximal end of the distal end constituting component (20).

3. The shaft according to Claim 2, wherein the distal end constituting component (20) of the guide tip (4) has insertion hole (22) that opens on the distal end and the side face of the proximal end and through which the guide wire is inserted.

4. The shaft according to Claim 2 or 3, wherein the proximal end constituting component (21) has a stepped shape with respect to the distal end constituting component (20), and the distal end constituting component (20) of the guide tip (4) has a proximal end face on the proximal end side.

5. The shaft according to any one of Claims 1 to 4, wherein the blood thrombus capturing member (3) has:
spindle-shaped wire member (8) around which a plurality of wires (13) are wound in a spiral, whose distal end component and proximal end component are constricted inward in the radial direction, and whose middle component bulges outward in the radial direction; and
a filter (11) that has a hole (17) and is externally fitted and fixed to the approximately one-half of the wire member (8) on the distal end side.

6. A blood thrombus capturing catheter, comprising:
a blood thrombus capturing member delivery sheath (1);
the shaft for operating a blood thrombus capturing member (2) according to any one of Claims 1 to 5; and
a blood thrombus capturing member (3) that is housed in the distal end of the sheath (1) and that protrudes forward in the axial direction of the sheath (1) by the operation of the shaft (2) and expands outward in the radial direction and contracts in the axial direction through elastic deformation.

7. The blood thrombus capturing catheter according to Claim 6, wherein the shaft (2) is equipped with a contact component (34), and the blood thrombus capturing catheter further comprises a rearward movement limiter (36) that comes into contact with the contact component (34) to limit rearward movement of the shaft (2) in the axial direction.

8. The catheter according to Claim 7, further comprising a forward movement limiter (37) that comes into contact with the contact component (34) to limits forward movement of the shaft (2) in the axial direction.

9. The catheter according to Claim 8, wherein the contact component (34) is externally fitted to a proximal end of the shaft (2) so as to be immovable in the axial direction, and
the blood thrombus capturing catheter further comprises a stopper (35) that is equipped on the proximal end of the shaft (2), and is cylindrical in shape, with both sides in the axial direction closed off, in which the proximal end of the shaft (2) is inserted movably in the axial direction, the contact component (34) is housed movably in the axial direction, and the proximal end serves as the rearward movement limiter (36), and the distal end serves as the forward movement limiter (37).

10. The catheter according to any one of Claims 6 to 9, wherein the shaft (2) is inserted movably in the axial direction into the blood thrombus capturing member delivery sheath(1), and protrudes forward from the distal end of the sheath (1).

11. The catheter according to any one of Claims 6 to 10, wherein the proximal end constituting component (21) has a stepped shape with respect to the distal end constituting component (20), and the distal end constituting component (20) of the guide tip (4) has a proximal end face on the proximal end side, and
when the blood thrombus capturing member (3) is housed in the blood thrombus capturing member delivery sheath (1), the proximal end constituting component (21) of the guide tip (4) is inserted into the distal end of the sheath (1), and a proximal end face of the distal end constituting component (21) of the guide tip (4) comes into contact with a distal end face of the sheath (1) to block off a distal end of the sheath (1).

12. The catheter according to any one of Claims 6 to 10, wherein the proximal end constituting component (21) of the guide tip (4) is inserted into the distal end of the sheath (1), and a side face of the distal end constituting component (21) of the guide tip (4) comes into contact with a inside face on the distal end side of the sheath (1) to block off a distal end of the sheath (1) when the blood thrombus capturing member (3) is housed in the blood thrombus capturing member delivery sheath (1).
